# EUROPEAN PATENT APPLICATION

(11) **EP 4 732 896 A1**
(43) Date of publication of application: **29.04.2026**
(21) Application number: 25210093.8
(22) Date of filing: 21.10.2025
(51) Int. Cl.: A61N 5/10, A61G 13/00

(54) **RADIOTHERAPY COUCH TOP**

(30) Priority: 25.10.2024 GB 202415779; 25.10.2024 GB 202415783; 11.11.2024 GB 202416552
(71) Applicant: Elekta Limited, Crawley, Sussex RH10 9RR (GB)
(72) Inventor: ALEXIS, Henrik, Crawley (GB); ERIKSSON, Markus, Crawley (GB)
(74) Representative: Thomas, Tomos Daniel

(57) **Abstract**

Disclosed herein is a radiotherapy couch top configured to support a subject thereon and comprising: a first lateral width in a first longitudinal region of the radiotherapy couch top; a second lateral width in a second longitudinal region of the radiotherapy couch top, the second lateral width being smaller than the first lateral width; and a curved lower surface.

## Description

This disclosure relates to a radiotherapy couch top, and in particular to a radiotherapy couch top with a curved lower surface. This disclosure also relates to a patient support apparatus and a radiotherapy device including the radiotherapy couch top.

### Background

Radiotherapy can be described as the use of ionising radiation, such as X-rays, to treat a human or animal body. Radiotherapy is commonly used to treat cancer, for example to treat tumours within the body of a patient or subject. In such treatments, ionising radiation is used to irradiate, and thus destroy or damage, cells which form part of the tumour.

Imaging can be performed to determine a position of a subject and of anatomical features within the subject. Such imaging can be performed before radiotherapy treatment begins with the subject in a treatment position, and/or can be performed while radiotherapy treatment is occurring. This helps ensure that the radiation is directed at the intended location, i.e. the location of the tumour, and not directed at other locations, e.g. locations of organs at risk. Radiotherapy treatment can be paused or adjusted if it is determined that the tumour is receiving or will receive a lower dose than would be desirable and/or if it is determined that healthy tissue is receiving or will receive a higher dose than would be desirable. By way of example, kV imaging (or MV imaging) can be used to provide images for these purposes.

It is desirable for the images produced by the kV imaging to be of high quality/high accuracy and to have as few artefacts as possible. This helps ensure that determinations made regarding locations of anatomical features of the subject, and the doses these anatomical features receive, are as accurate as possible. However, a subject is typically disposed on a patient couch during such imaging and during such radiotherapy treatment. In order to image the subject, the imaging beam (and the treatment beam) typically passes through the patient couch as well as through the subject. Properties of the patient couch can therefore introduce artefacts into the images produced, which limits the accuracy with which the subject themselves can be imaged.

In view of the above, it would be desirable to increase the quality and accuracy of imaging for radiotherapy and to reduce the artefacts present in images generated using such imaging. Via these means, it would be desirable to increase the safety and accuracy of radiotherapy treatments.

The present invention seeks to address these and other disadvantages encountered in the prior art.

### Summary

An invention is defined in the independent claims. Optional features are set out in the dependent claims.

### Figures

Specific embodiments are now described, by way of example only, with reference to the drawings, in which:
Figure 1 depicts a radiotherapy device or apparatus according to the present disclosure;
Figure 2 depicts a patient support apparatus according to the present disclosure;
Figure 3 depicts a top-down view of a couch top according to the present disclosure;
Figure 4 depicts a cross-section of a couch top according to the present disclosure;
Figure 5 depicts a further cross-section of a couch top according to the present disclosure;
Figure 6 depicts a further cross-section of a couch top according to the present disclosure.

### Detailed Description

In overview, and without limitation, the application relates to a couch top for supporting a subject. The couch top can be implemented as part of a patient support apparatus and can be used to support the subject within a radiotherapy device. The couch top includes a first portion and a second portion at different points along the length of the couch top, with the second portion being narrower in width than the first portion. In other words, the couch top includes a wider first portion, which can be used to support a torso of a subject, and a narrower second portion, which can be used to support a head of the subject. The lower surface of the couch top is curved. In other words, when moving from a first lateral side of the couch top to a second lateral side of the couch top, the lower surface of the couch top follows a curved shape such that its vertical height above the ground differs when moving from this first lateral side to this second lateral side.

Radiotherapy couch tops need to be stiff enough such that they don't bend too much when a subject is disposed on the couch top, which can lead to the subject being in an unintended or suboptimal position for a planned treatment. It is also desirable for a radiotherapy couch top to be relatively thin in order to limit attenuation of the treatment beam by the couch top and not take up too much space in the treatment area. In addition, it is desirable to provide a flat upper surface since this is typically required for compatibility with attachment of accessories and fixation devices thereto. These requirements are typically met by providing radiotherapy couch tops which are flat/square/rectangular in profile.

However, it has been found that the flat profile of the couch top results in substantial imaging artefacts, particularly for kV imaging beam angles which are parallel or close to parallel to the top or bottom surface of the couch top (i.e. when the imaging beam is generally horizontal). This is because from this orientation the beam can pass through the whole width of the couch top, or not, dependent on small movements in angle/position. Moreover, a finite number of kV images may be generated at a finite number of angular positions and the intensity resolution and spatial resolution of the kV detector, and the gantry angles at which the images are generated, will all have errors/uncertainties. This can cause further difficulties with imaging through a 'cliff-edge' in the presence of absence of substantial couch top material. In other words, for small variations in angle, the measured projection images of the couch top will have a large and sharp variation. This can be difficult to resolve, giving streak artefacts in the generated images. While these artefacts may be at an acceptable level for certain imaging and certain treatments, it would be desirable to facilitate higher quality imaging with fewer artefacts in order to more accurately determine the locations of anatomical features of the subject at different times. This in turn can be used to support and inform safer radiotherapy treatments.

As described herein, the proposed couch top has a curved lower surface. This reduces the number/severity of artefacts in the images generated such that more reliable imaging, and consequently more reliable image-guided treatment, can be provided. The above-mentioned streak artefacts may be less numerous/less severe with the curved surface because there is a less sharp variation between the beam passing through substantial couch top material and no couch top material.

Moreover, the curved underside of the couch top may be shaped to correspond to the curved interior of a bore of the radiotherapy device which the couch top is implemented in. This may enable more optimal use of the available space within the bore. The curved shape of the couch top may also reduce vertical deflection of the couch top when the subject is disposed thereon, relative to the situation with a laminar couch top. This may enable more accurate imaging and treatment of the subject without major positional errors, particularly of locations distant from the support base which supports the couch top. This in turn enables long treatment fields and treatment of the full table stroke length in one treatment plan/treatment session.

Accordingly, disclosed herein is a radiotherapy couch top configured to support a subject thereon and comprising: a first portion in a first longitudinal region of the radiotherapy couch top; a second portion in a second longitudinal region of the radiotherapy couch top, a lateral width of the second portion being smaller than a lateral width of the first portion; and a curved lower surface.

As discussed herein, the curved lower surface enables more accurate imaging of the subject to be provided. In addition, the narrower second longitudinal region can be used to support a head of a subject without large amounts of couch top material extending either side of the head of the subject. This enables a treatment beam direct access to the head of the subject, without also passing through the couch top, from a greater range of angles, which would otherwise attenuate the treatment beam. This is of particular importance for brain treatments since it is particularly important to be accurate for these treatments. In addition, since the curved lower surface increases the thickness of the couch top to some extent, this would increase the attenuation through the couch top such that providing this narrower width of couch top for the head becomes particularly important. Therefore, the combination of features of the couch top interrelate to enable more accurate imaging and more accurate radiotherapy treatment to be simultaneously achieved.

Optionally, the radiotherapy couch top comprises first and second lateral sides, wherein the curved lower surface forms a curved shape extending from the first lateral side to the second lateral side. Optionally, the curved lower surface is curved with respect to a lateral axis of the radiotherapy couch top. Optionally, the curved lower surface is convex with respect to the lateral axis of the radiotherapy couch top. Optionally, the curved lower surface is curved such that a lateral centre of the curved lower surface is at a lower vertical position than lateral edges of the curved lower surface. Optionally, the curved lower surface is symmetrical with respect to a longitudinal axis of the radiotherapy couch top.

Optionally, the curved lower surface has a single, constant radius of curvature. Optionally, the radius of curvature is in the range of 400 mm to 2000 mm.

Optionally, the curved lower surface comprises a plurality of sub-sections each with a different respective radius of curvature. Optionally, the different respective radii of curvature are in the range of 200 mm to 2000 mm.

Optionally, the first longitudinal region of the radiotherapy couch top is configured to support a head of the subject. Optionally, the second longitudinal region of the radiotherapy couch top is configured to support a torso of the patient.

Optionally, the radiotherapy couch top comprises a third longitudinal region of the radiotherapy couch top between the first longitudinal region and the second longitudinal region, wherein the radiotherapy couch top has a third lateral width in the third longitudinal region which decreases from being equal to the first lateral width of the first longitudinal region at an end of the third longitudinal region which is adjacent to the first longitudinal region, to being equal to the second lateral width of the second longitudinal region at an end of the third longitudinal region which is adjacent to the second longitudinal region.

Optionally, a curvature of the curved lower surface in the first longitudinal region is different to a curvature of the curved lower surface in the second longitudinal region. Optionally, a radius of curvature of the curved lower surface in the second longitudinal region is smaller than a radius of curvature of the curved lower surface in the first longitudinal region. Optionally, the radius of curvature of the curved lower surface in the second longitudinal region is in the range of 250 mm to 1000 mm.

Optionally, the radiotherapy couch top comprises a flat upper surface.

Optionally, the radiotherapy couch top comprises a curved upper surface. Optionally, the curved upper surface is curved such that a lateral centre of the curved upper surface is at a lower vertical position than lateral edges of the curved upper surface. Optionally, a curvature of the curved upper surface is different to a curvature of the curved lower surface. Optionally, a thickness of the radiotherapy couch top between the curved upper surface and the curved lower surface varies laterally across the radiotherapy couch top. Optionally, the curved upper surface has a radius of curvature in the range of 1500 mm to 2500 mm.

Also disclosed herein is a patient support apparatus comprising: the radiotherapy couch top as described above; and a patient support base configured to support the radiotherapy couch top.

Also disclosed herein is a radiotherapy device comprising: a rotatable gantry; a radiation source; an imaging apparatus; and the patient support apparatus as described above.

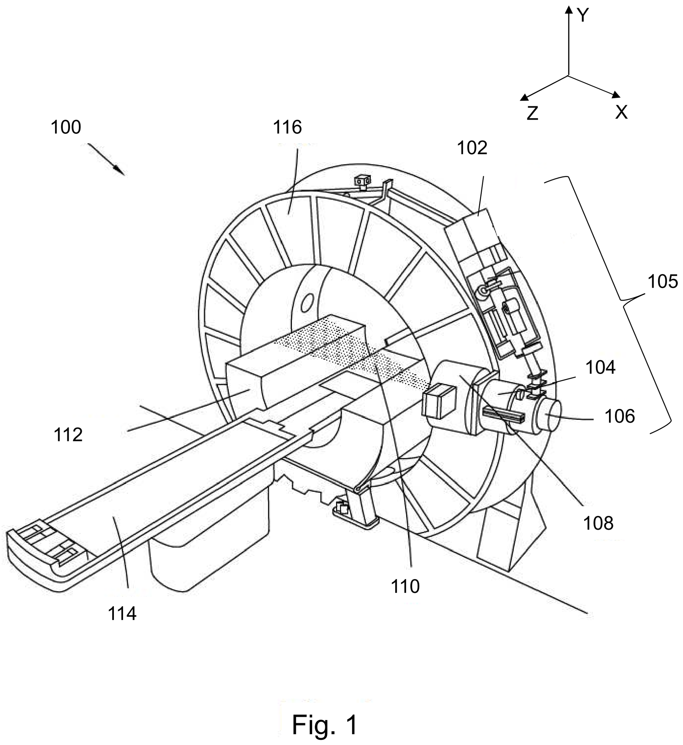

Figure 1 depicts a radiotherapy device 100. The radiotherapy device 100 is suitable for delivering, and configured to deliver, a beam of radiation 110 to a patient during radiotherapy treatment. The device 100 and its constituent components will be described generally for the purpose of providing useful accompanying information for the present application. The device 100 depicted in Figure 1 is in accordance with the present disclosure and is suitable for use with the disclosed systems and apparatuses. While the device 100 in Figure 1 is an MR-linac, in implementations of the present disclosure the device 100 may be another type of radiotherapy device, for example a linac device with a different imaging capability.

In overview, the radiotherapy device 100 comprises a source of radiation 105 and an imaging apparatus 112. The source of radiation 105 is coupled to a rotatable gantry 116. The device 100 comprises a patient positioning apparatus which comprises a patient positioning surface 114 on which a patient may be positioned. Before treatment, the patient is positioned on the surface 114, and the patient positioning apparatus may be used to position the patient in an appropriate position for the treatment, for example according to a reference image on which the patient's treatment plan is based. During treatment, the source of radiation 105 delivers radiation to the patient according to the patient's treatment plan.

The source of radiation 105 is configured to generate a beam of radiation 110, and in particular a beam of therapeutic radiation. The radiation source 105 is attached to the rotatable gantry 116 so as to rotate with the gantry 116. In this way, the radiation source is rotatable around the patient so that the beam 110 can be applied from different angles around the gantry 116. The source of radiation 105 may comprise a beam generation system comprising a linear accelerator (linac). For such a linac device, the beam generation system may comprise a source of RF energy 102, an electron gun 106, and a waveguide 104.

The source 102 of radiofrequency waves, such as a magnetron, is configured to produce radiofrequency waves. The source 102 of radiofrequency waves is coupled to the waveguide 104, for example via a circulator, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source 106 and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide 104 typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun 106. Electrons can be accelerated to speeds approaching the speed of light in the evacuated waveguide 104.

Once the electrons have been accelerated, they travel toward a heavy metal target which, when impacted by the electrons, generates a beam of high energy photons, forming a radiation beam 110. When the electrons strike the target, X-rays are produced in a variety of directions. The device 100 comprises collimation apparatus 108. The collimation apparatus 108 may comprise a primary collimator. The primary collimator is configured to block X-rays travelling in certain directions and pass only forward travelling X-rays to produce a treatment beam 110. The X-rays may be filtered and may pass through one or more ion chambers for dose measuring. The collimation apparatus 108 may additionally comprise beam shaping apparatus such as a multi-leaf collimator (MLC). The beam can be shaped in various ways by the beam-shaping apparatus. The source of radiation is configured to direct the beam 110 of therapeutic radiation, having been appropriately filtered and shaped by the collimation apparatus 108, toward a patient positioned on the patient support surface 114.

The device 100 comprises an imaging apparatus 112 or 'image acquisition apparatus'. The depicted imaging apparatus 112 is an MR imaging apparatus, though the imaging apparatus may take other forms, for example a cone beam computed tomography (CBCT) apparatus. The MR imaging apparatus 112 is shown in cross-section in the diagram. The imaging apparatus 112 is configured to generate imaging data. The imaging data may comprise images of the patient. The imaging apparatus 112 is therefore configured to obtain images of a patient positioned on the patient support surface 114. The imaging data generated by the imaging apparatus 112 may be used to generate a reference image to enable treatment planning, and/or may be used during the delivery of therapeutic radiation to help guide the beam of radiation 110 or to provide an input into motion management and real-time adaptive radiotherapy techniques.

As described herein, the radiotherapy device 100 may comprise an imaging apparatus 112 which is a kV imaging apparatus. While the skilled person will be familiar with kV imaging apparatuses, the following brief description is provided. The kV imaging apparatus may comprise a kV beam source and a kV detector, each of which may be fixed to the rotatable gantry opposite (at 180° to) each other. Each of the kV beam source and the kV detector may be offset by 90° relative to the source of radiation 105. The kV beam source may be configured to generate a kV X-ray beam directed through the subject (and in general through the patient support surface 114), to be incident on the kV detector. The kV detector may be configured to generate an image of the subject based on the kV X-ray beam it receives, as attenuated by the subject (and in general the patient support surface 114) which the kV X-ray beam passes through.

Figure 1 and its accompanying description herein is provided to give context to the application and to facilitate understanding of the present invention. In addition to the components described in overview above, a radiotherapy device also comprises many other functions, components and subsystems as will be understood by the skilled person.

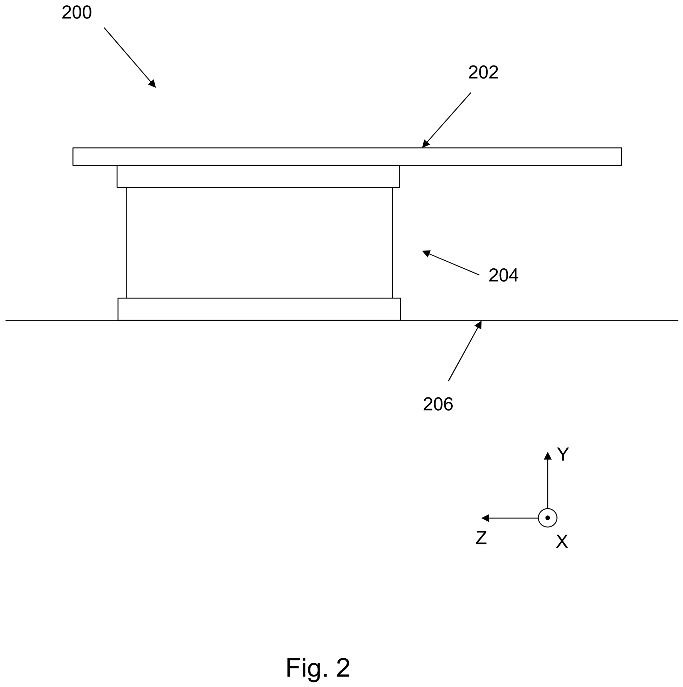

Figure 2 depicts a schematic of a patient support apparatus 200 according to the present disclosure.

The patient support apparatus 200 comprises a couch top 202, i.e. a radiotherapy couch top 202. The patient support apparatus 200, or the couch top 202, may correspond to the patient support apparatus 114 described in relation to Figure 1. The couch top 202 is configured to support a subject, i.e. a patient, thereon. In other words, in use, a subject may be disposed on and in contact with an upper surface of the couch top 202. The couch top 202 may be stiff/rigid enough such that it does not bend or sag significantly when a subject is disposed thereon. The couch top may comprise a foam core with a fibre skin. The fibre skin can comprise, by way of example, carbon fibres or aramid fibres or hemp fibres. These fibre materials may not cause excessive attenuation of the imaging beam. For example, the use of carbon minimises the attenuation of the imaging and treatment beams because its low atomic number means that each carbon atom has relatively few electrons. While the couch top 202 is depicted schematically as being generally flat in the side-on view of Figure 2 for ease of illustration, the present disclosure provides that a couch top 202 has a curved lower surface, as well as first and second lateral widths in first and second longitudinal regions respectively.

The patient support apparatus 200 also comprises a patient support base 204. The patient support base 204 is configured to support the couch top 202. The patient support base 204 may be disposed on a floor 206 of a room housing the patient support apparatus 200/radiotherapy device 100. The patient support base 204 may comprise one or more drivers, actuators and/or other mechanical/electrical components configured to cause movement of the couch top 202. This movement may comprise linear movement along three perpendicular axes, as well as rotational movement around each of these three perpendicular axes. In other words, the patient support apparatus 200 may be configured for 6D movement, though in some examples the patient support apparatus 200 may not be configured to provide movement along/around one or more of these directions, e.g. may be configured for 3D, 4D or 5D movement.

The couch top 202 may be fixed to the patient support base 204 such that tilting or translation of all or part of the patient support base 204 causes tilting or translation of the couch top 202. This may be used to position the couch top 202, and the subject thereon, into a desired position for imaging and/or radiotherapy. The couch top 202 may be slidably coupled to the patient support base 204, enabling linear movement of the couch top 202 relative to the patient support base 204 (e.g. along the Z-axis as depicted in Figure 2). In some examples, some of the driving electronics or mechanical features may be couped to or integrated into the couch top 202, in a region of the couch top 202 (an 'attachment zone') which is not irradiated by treatment or imaging beams. One or more attachment blocks may be provided (in the attachment zone) between the couch top 202 and the patient support base 204. For example, four such attachment blocks may be provided. The one or more attachment blocks may be configured to couple the couch top 202 to the patient support base 204.

The patient support base 204 may at least be configured to cause the couch top to translate along a left-right direction as depicted in Figure 2 (parallel to the Z-axis). Other components of the radiotherapy apparatus 100 of Figure 1, such as the source of radiation 105 and the imaging apparatus 112, may generally be located to the right of the patient support apparatus 200 as depicted in Figure 2. In a 'setup position' which enables a subject to easily mount the couch top 202, the couch top 202 may be translated/disposed towards the left of Figure 2. The couch top 202 may be translated/disposed to the right as depicted in Figure 2, i.e. towards the other components of the radiotherapy apparatus 100, to place the subject in a 'treatment position' in which they can be imaged and/or treated. The couch top 202 may for example be translated in this manner using a linear guide or linear rail of the patient support base 204 coupled to the couch top 202.

The present application focuses on the form of the couch top 202 as described herein. It will be appreciated that this couch top 202 is generally applicable to various different forms of patient support base 204 and patient support apparatus 200 without limitation, and that the depiction and discussion of Figure 2 is provided merely as an example for understanding the context of the current application. The couch top 202 is the part of the patient support apparatus 200 disposed to contact the subject and configured for support of the subject, in contrast to the parts of the patient support apparatus 200 configured to control/drive movement of the couch top 202 with the subject thereon.

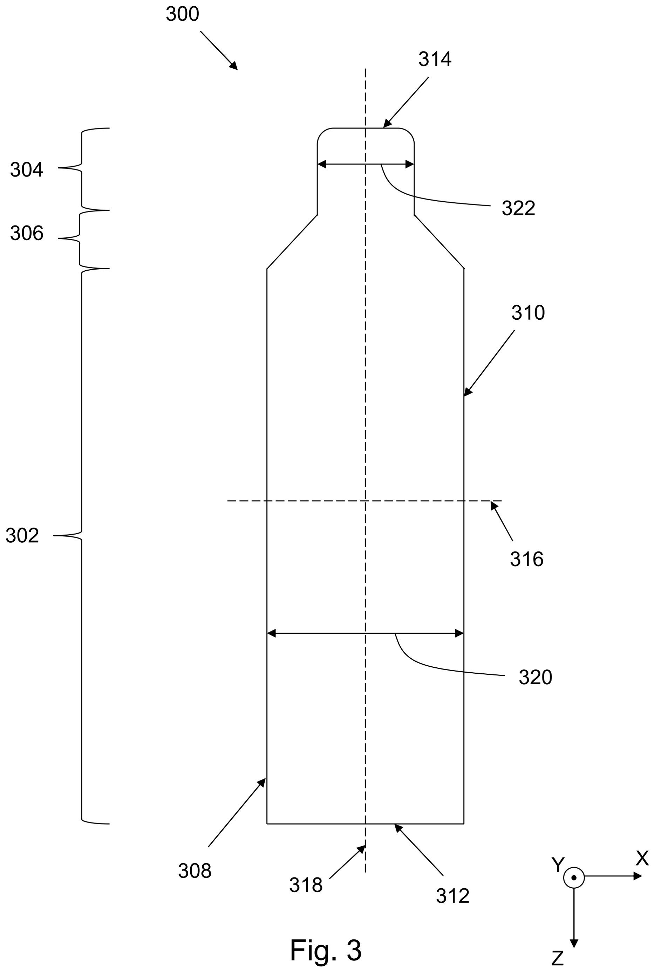

Figure 3 depicts a top-down view of a couch top 300 according to the present disclosure. The couch top 300 may correspond to the couch top 202 described in relation to Figure 2.

The couch top 300 comprises a first longitudinal region 302 and a second longitudinal region 304. In other words, the couch top 300 comprises a first portion in a first longitudinal region 302 of the couch top 300 and a second portion in a second longitudinal region 304 of the couch top 300.The couch top 300 may also comprise a third longitudinal region 306, i.e. a third portion in a third longitudinal region 306 of the couch top, between the first longitudinal region 302 and the second longitudinal region 304.

The couch top 300 may comprise a first lateral side 308 and a second lateral side 310. The couch top 300 may comprise a first longitudinal end 312 and a second longitudinal end 314. The couch top 300 may extend between the first lateral side 308 and the second lateral side 310, each of which may be described as being disposed at different ends of a lateral axis 316 of the couch top 300. The couch top 300 may extend between the first longitudinal end 312 and the second longitudinal end 314, each of which may be described as being disposed at different ends of a longitudinal axis 318 of the couch top 300. The longitudinal axis 318 may be perpendicular to the lateral axis 306. The first and second lateral sides 308, 310 may be parallel to the longitudinal axis 318. The first and second longitudinal ends 312, 314 may be parallel to the lateral axis 316.

The first longitudinal region 302 of the couch top 300 has a first lateral width 320. This lateral width is a width of the couch top 300 along the lateral axis 316 of the couch top 300 between the first lateral side 308 and the second lateral side 310. The couch top 300 may have the first lateral width 320 throughout or in most of the first longitudinal region 302. The second longitudinal region 304 of the couch top 300 has a second lateral width 322. This lateral width is a width of the couch top 300 along the lateral axis 316 of the couch top 300 between the first lateral side 308 and the second lateral side 310. The couch top 300 may have the second lateral width 322 throughout or in most of the second longitudinal region 304.

The second lateral width 322 of the couch top 300 in the second longitudinal region 304 is smaller than the first lateral width 320 of the couch top in the first longitudinal region 302. In other words, the first longitudinal region 302 is wider than the second longitudinal region 304. The second longitudinal region 304 is narrower than the first longitudinal region 302. The couch top 300 is not of uniform width along its length (along the longitudinal axis 318 thereof), but instead has a wider portion along a first part of its length and a narrower portion along a second part of its length.

The first longitudinal region 302 is at or adjacent to the first longitudinal end 312. The first longitudinal region 302 is disposed closer to the first longitudinal end 312 than the second longitudinal region 304 is. The first longitudinal region 302 may comprise an attachment zone configured to couple the couch top 300 to the patient support base 204 of the patient support apparatus 200 (see Figure 2). The attachment zone may be disposed adjacent to the first longitudinal end 312, i.e. at the bottom of Figure 3. The first longitudinal region 302 may comprise a body treatment zone configured to support a torso of a subject disposed on the couch top 300. The body treatment zone may be disposed closer to the second longitudinal region 304 than the attachment zone is. The body treatment zone may be disposed between the attachment zone and the second longitudinal region 304 or between the attachment zone and the third longitudinal region 306. In use, the couch top 300 may be positioned in relation to other components of the radiotherapy device 100 such that imaging and/or therapeutic radiation passes through the body treatment zone but not through the attachment zone.

The second longitudinal region 304 is at or adjacent to the second longitudinal end 314. The second longitudinal region 304 may be comprise or correspond to a head treatment zone configured to support a head of a subject disposed on the couch top 300. In use, imaging and/or therapeutic radiation passes through the head treatment zone. In view of the relative sizes of human torsos and heads, the second longitudinal region 304 may be shorter (along the longitudinal axis 318) than the first longitudinal region 302 and is narrower (along the lateral axis 316) than the first longitudinal region 302.

Since the second longitudinal region 304 has the narrower second lateral width 322, the head of the subject is more exposed in that a radiotherapy beam can be directed through the head of the subject without also having to pass through the couch top 300 from a greater range of positions/angles of the source of radiation 105. This may increase the accuracy, efficiency and/or versatility of head treatments. Typically, radiotherapy is applied with the source of radiation 105 located at various angles around the subject using the rotatable gantry 116. It will be appreciated that, if the couch top 300 had the same, first longitudinal width along its whole length, the radiotherapy beam would have to pass through the couch top 300 for irradiation with the source of radiation 105 positioned in at least the lower 180° of the circle described by the rotatable gantry 116. However, since the second longitudinal region 304 is provided with the smaller, second lateral width 322, depending on the particular anatomical target location within the head, this enables direct irradiation of the target without the radiotherapy beam also passing through the couch top 300 with the source of radiation 105 at a greater range of angles. For example, this may be enabled with the source of radiation 105 substantially to the left or right of the couch top 300 (along the X-axis), but below the height of the couch top 300 to some extent (along the Y-axis). This increases the scope for generating treatment plans and corresponding treatments which more accurately target particular locations and shapes of tumours. Moreover, the narrower second lateral width 322 may prevent the kV imaging detector/panel from colliding with the couch top when it rotates.

The above discussion has focused on the subject being reclined with their head disposed in the second longitudinal region 304 and their torso disposed in the first longitudinal region 302. The subject may be disposed on their back with their back in contact with an upper surface of the couch top 300. Alternatively, the subject may be disposed on their front with their front in contact with the upper surface of the couch top 300. Moreover, in some examples the subject may be disposed on the couch top in other ways. For example, in 'feet first' treatments, the subject may be disposed with their head adjacent to the first longitudinal end 312 and their feet adjacent to the second longitudinal end 314.

An extension or cover board configured to couple to the second longitudinal portion 304 of the couch top 300 may be provided. The extension may have a lateral width equal or similar to the first lateral width 320 of the first longitudinal portion 302 of the couch top 300. The extension may be used for better supporting the feet/legs of the subject in feet first treatments and may be used for better supporting the arms of the subject in some treatments in which the subject is lying on their front.

The third longitudinal region 306 may couple the first longitudinal region 302 to the second longitudinal region 304. The third longitudinal region 306 may have a third lateral width which varies along its length. The third lateral width may be equal to the first lateral width 320 of the first longitudinal region 302 at an end of the third longitudinal region 306 that is adjacent to the first longitudinal region 302. The third lateral width may be equal to the second lateral width 322 of the second longitudinal region 304 at an end of the third longitudinal region 306 that is adjacent to the second longitudinal region 304.

In other words, the third lateral width may decrease along the longitudinal axis 318 of the couch top 300 when moving towards the second longitudinal end 314 and away from the first longitudinal end 312. The rate of this decrease in width may be substantially constant. In other words, the first and second lateral sides 308, 310 of the couch top in the third longitudinal region 306 may be straight lines. The first and second lateral sides 308 of the couch top in the third longitudinal region 306 may be (diagonally) angled with respect to the longitudinal axis 318, e.g. may form an approximately 45° angle with the longitudinal axis 318.

The third longitudinal region 306 may provide a gradual transition between the first longitudinal region 302 and the second longitudinal region 304. Considering the dose applied to a subject in a radiotherapy treatment, whether or not the radiotherapy beam passes through the couch top 300 or not can significantly affect the dose received by the subject. As such, when there is a sharp cut off between presence and absence of the couch top 300, small positional errors bridging the cut off can lead to large dosimetric errors. The gradual transition provided by the third longitudinal region 306 can reduce the magnitude of such dosimetric errors resulting from small positional errors.

The first longitudinal region 302 may be integrally formed with the second longitudinal region 304. The first longitudinal region 302 may be integrally formed with the third longitudinal region 306 and the third longitudinal region 306 may be integrally formed with the second longitudinal region 304. The different portions of the couch top 300 may be described as being integrally formed, being formed as one structure, being monolithic, and/or being formed without joints therebetween. This can help prevent such joints causing artefacts in the images produced of the subject and/or can help prevent dosimetric discrepancies.

While the couch top 300 of Figure 3 has been provided by way of example, various different forms of couch top may be used according to the present disclosure. In some examples, the couch top may have a constant width along its length, i.e. along the whole longitudinal axis 318 thereof. In other words, the couch top may be rectangular/substantially rectangular in the X-Z plane.

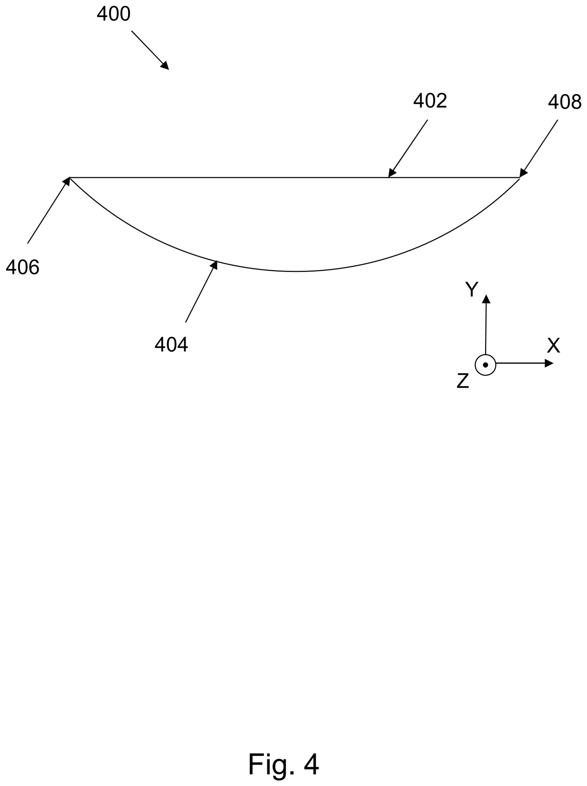

Figure 4 depicts a cross-section of a couch top 400 according to the present disclosure. The couch top 400 may correspond to the couch tops 202 and 300 described in relation to Figures 2 and 3 respectively. The cross-section depicts a plane of the couch top 400 parallel to the lateral axis 316 of the couch top 400 at a point along the longitudinal axis 318 of the couch top 400 (see Figure 3).

The couch top 400 comprises an upper surface 402 and a lower surface 404. The lower surface 404 is curved, i.e. may be described as a curved lower surface 404. As depicted in Figure 4, the upper surface 402 may be flat or planar. The couch top 400 comprises a first lateral side 406 and a second lateral side 408, which may correspond respectively to the first and second lateral sides 308, 310 described in relation to Figure 3. In use, a subject may be disposed on and in contact with the upper surface 402 of the couch top 400.

The curved lower surface 404 may be described as curved with respect to the upper surface 402 and/or curved with respect to the lateral axis 316 of the couch top 400. The curve of the lower surface may extend substantially to the first and second lateral sides 406, 408 of the couch top 400.

The curved lower surface 404 may be convex, i.e. may be curved such that a lateral centre of the curved lower surface 404 is at a lower vertical position (along the Y-axis) than the first and second lateral sides 406, 408. The curved lower surface 404 may be symmetrical with respect to the longitudinal axis 318 of the couch top 400.

The curvature of the lower surface 404 enables minimisation of image artefacts in kV images generated with the couch top 400 in the field of view, particularly for imaging beams directed close to parallel to the lateral axis 316. As can be seen from Figure 4, the curved lower surface 404 means that the form of the couch top 400 is such that the thickness of the couch top 400 gradually increases when moving from a lateral side 406, 408 of the couch top 400 to a lateral centre of the couch top 400. Through avoiding an immediate transition between no couch top intersecting with an imaging beam and a uniform thickness of couch top intersecting with the imaging beam (as in couch tops with a rectangular cross-section), the couch top 400 of the present disclosure avoids imaging artefacts resulting from this discontinuity. Therefore, through implementation of the curved lower surface 404 in the couch top 400 described herein, the accuracy and reliability of images generated of a subject disposed on the couch top 400 can be improved.

As depicted in Figure 4, the curved lower surface 404 may have a single radius of curvature across substantially the whole of its lateral width. In other words, the curved lower surface 404 may have the shape of an arc of a circle (with a particular radius), which may provide a particularly stable form of couch top. In other examples, the curved lower surface 404 may have the shape of a parabola or another form of curve. The curved underside of the couch top may be shaped to correspond to the curved interior of a bore of the radiotherapy device which the couch top is implemented in. In other words, the radius of curvature of the lower surface of the couch top may be equal to or approximately equal to the radius of curvature of the interior of the bore. This may enable more optimal use of the available space within the bore.

The cross-section depicted in Figure 4 may be a cross-section through the first longitudinal region 302, though it may also represent a cross-section through the second longitudinal region 304 or the third longitudinal region 306. The cross-section and the curved lower surface 404 may be the same throughout the first longitudinal region 302. In other examples, the lower surface 404 may only be curved in the body treatment zone but not in the attachment zone (discussed in relation to Figure 3). In other words, the lower surface of the couch top 400 may be flat in the attachment zone to enable more mechanically simple/reliable coupling to the patient support base 204 since the attachment zone, unlike the body treatment zone, is not imaged such that providing the curved lower surface 404 in this zone may not be needed or advantageous.

The radius of curvature in the first longitudinal region 302 may be greater than or equal to 400 mm. The radius of curvature may be equal to or smaller than 2000 mm. This range is particularly advantageous since smaller radii of curvature may lead to a couch top 400 which is undesirably thick, while larger radii of curvature tend to a flat geometry in which reduction of imaging artefacts is not significantly achieved.

In the second longitudinal region 304, the cross-section may be substantially similar to that depicted in Figure 4. The radius of curvature of the curved lower surface 404 in the second longitudinal region 304 may be different to the radius of curvature in the first longitudinal region 302. The radius of curvature of the curved lower surface 404 in the second longitudinal region 304 may be smaller than the radius of curvature in the first longitudinal region 302. This is because the second longitudinal region 304 has a smaller lateral width 322 than the lateral width 320 of the first longitudinal region 302, such that a higher degree of curvature may be implemented in the second longitudinal region 304. The radius of curvature of the curved lower surface 404 in the second longitudinal region 304 may be greater than or equal to 250 mm. The radius of curvature of the curved lower surface 404 in the second longitudinal region 304 may be equal to or smaller than 1000 mm. For example, the radius of curvature of the curved lower surface 404 in the second longitudinal region 304 may be approximately 300 mm. This range may provide an advantageous trade-off between thickness and reduction of artefacts, as described above in relation to the first longitudinal region 302. The cross-section and the curved lower surface 404 may be the same throughout the second longitudinal region 304.

The combination of the curved lower surface 404 and the smaller lateral width 322 of the second longitudinal region 304 simultaneously enables direct irradiation of the head of a subject for a greater range of treatment angles and imaging with less severe artefacts in order to better inform such treatments. Provision of the different forms of curved lower surface 404 in the first longitudinal region 302 and the second longitudinal region 304 respectively further enables this curved lower surface to be implemented in an optimised manner for the different regions of the couch top 400.

In the third longitudinal region 306, the cross-section may also be substantially similar to that depicted in Figure 4. The curvature of the lower surface 404 in the third longitudinal region 306 may vary along the longitudinal axis of the couch top 400 such that a smooth transition is provided between the curved lower surface 404 of the first and second longitudinal regions 302, 304. The radius of curvature of the curved lower surface 404 in the third longitudinal region 306 may decrease from being equal to the radius of curvature of the curved lower surface 404 in the first longitudinal region 302 at a position adjacent to the first longitudinal region 302, to being equal to the radius of curvature of the curved lower surface 404 in the second longitudinal region 304 at a position adjacent to the second longitudinal region 304.

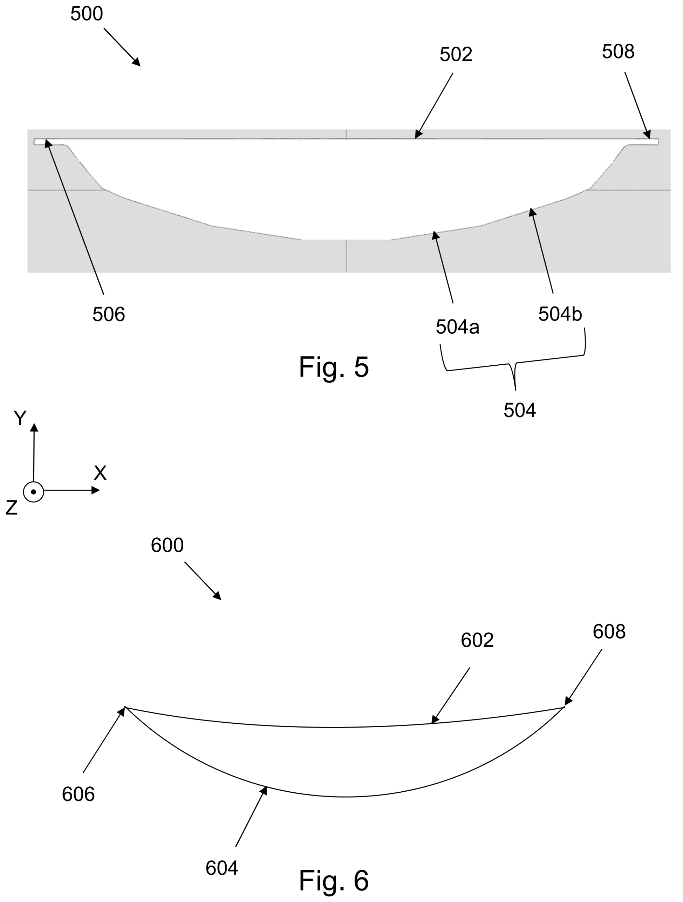

Figure 5 depicts a further cross-section of a couch top 500 according to the present disclosure. The couch top 500 may correspond to the couch tops 202 and 300 described in relation to Figures 2 and 3 respectively. The cross-section depicts a plane of the couch top 500 parallel to the lateral axis 316 of the couch top 500 at a point along the longitudinal axis 318 of the couch top 500 (see Figure 3). The couch top 500 may have corresponding features to the couch top 400 described in relation to Figure 4 except where indicated otherwise below.

The couch top 500 comprises an upper surface 502, and a curved lower surface generally depicted using reference numeral 504. The couch top 500 comprises first and second lateral sides 506, 508. In Figure 5, the body of the couch top 500 is represented by the white space in the centre of the image, while the grey space around the couch top 500 represents empty space around the couch top 500.

In contrast to the couch top 400 of Figure 4, the curved lower surface 504 of couch top 500 comprises a plurality of sub-sections 504a, 504b, each with a different respective radius of curvature and each located in a different respective area along the lateral axis 316 of the couch top 500. While only two of these are labelled in Figure 5 for ease of illustration, it will be appreciated that the curved lower surface 504 may comprise any number of these sub-sections. The respective radii of curvature of the sub-sections 504a, 504b may be greater than or equal to 200 mm and equal to or smaller than 2000 mm. Radii of curvature of sub-sections closer to the lateral centre of the couch top 500 may be larger than radii of curvature of subsections closer to the lateral sides 506, 508. This may provide the curved form of the couch top 500 enabling reduction of imaging artefacts while increasing the thickness of the couch top 500 less than would be the case for a curved lower surface 504 with a single radius of curvature.

The plurality of sub-sections 504a, 504b may extend substantially to the lateral sides 506, 508 of the couch top 500. As depicted, and as applies throughout this disclosure, in some examples narrow regions at the lateral edges of the couch top 500 may not have curved upper/lower surfaces 502, 504, but instead may have flat upper and lower surfaces 502, 504, between which the couch top 500 may be relatively thin (in the Y-direction). These may facilitate easier attachment of the couch top 500 to a support base.

The cross-section depicted in Figure 5 may be a cross-section through the first longitudinal region 302, the second longitudinal region 304 or the third longitudinal region 306. The cross-section may have the same form throughout the first longitudinal region 302, or may have a flat lower surface in an attachment zone of the first longitudinal region 302 as described in relation to Figure 4. The cross-section may have the same form throughout the second longitudinal region 304. The cross-section in the second longitudinal region 304 may differ from the cross-section in the first longitudinal region 302. For example, the lower surface 504 may generally have a higher degree of curvature in the second longitudinal region 304 than in the first longitudinal region 302 through the radii of curvature of the subsections 504a, 504b being smaller and/or through a different number of such subsections 504a, 504b being present. The cross-section in the third longitudinal region 306 may vary along the longitudinal axis 318 of the couch top 500 so as to provide a smooth transition between the first longitudinal region 302 and the second longitudinal region 304.

Figure 6 depicts a further cross-section of a couch top 600 according to the present disclosure. The couch top 600 may correspond to the couch tops 202 and 300 described in relation to Figures 2 and 3 respectively. The cross-section depicts a plane of the couch top 600 parallel to the lateral axis 316 of the couch top 600 at a point along the longitudinal axis 318 of the couch top 600 (see Figure 3). The couch top 600 may have corresponding features to the couch tops 400, 500 described in relation to Figures 4 and 5 respectively except where indicated otherwise below.

The couch top 600 comprises an upper surface 602, a lower surface 604 and first and second lateral sides 606, 608. As depicted in Figure 6, the lower surface 604 is curved, i.e. is a curved lower surface 604. As depicted in Figure 6, the upper surface 602 is curved, i.e. may be a curved upper surface 602. The upper surface 602 may be curved along/relative to the lateral axis 316 between the first and second lateral sides 606, 608. Providing the curved upper surface 602 is particularly advantageous for reducing imaging artefacts since it is closer to the region of interest of the imaging and of the radiotherapy, i.e. close to anatomical regions within the body of the subject.

The curved upper surface 602 may be curved relative to the curved lower surface 604. In other words, the curved upper surface 602 may not be parallel to the curved lower surface 604, but instead may have a different curvature thereto. The curved upper surface 602 may have a larger radius of curvature than the curved lower surface 604. The radius of curvature of the curved upper surface 602 may be greater than or equal to 1500 mm. The radius of curvature of the curved lower surface 602 may be equal to or less than 2500 mm.

Providing the different (lesser) curvature of the upper surface 602 may advantageously increase comfort of the subject when positioned on the couch top 600. Patient positioning devices and other accessories for radiotherapy are typically constructed to be fixed to flat surfaces. When implemented with the couch top 600 of Figure 6, such patient positioning devices/accessories may be formed with a specific curvature to correspond to the curvature of the upper surface 602, or an infill may be provided to fit between patient positioning devices/accessories and the upper surface 602 or to couple to an indexing bar.

In some examples, the couch top 600 may comprise a lower surface 604 with a plurality of sub-sections each with a different respective radius of curvature, as described in relation to Figure 5. In some examples, the couch top 600 may comprise an upper surface 602 with a plurality of such sub-sections each with a different respective radius of curvature.

The cross-section depicted in Figure 6 may be a cross-section through the first longitudinal region 302, the second longitudinal region 304 or the third longitudinal region 306. The cross-section may have the same form throughout the first longitudinal region 302, or may have a flat lower surface in an attachment zone of the first longitudinal region 302 as described in relation to Figure 4. The cross-section may have the same form throughout the second longitudinal region 304. The cross-section in the second longitudinal region 304 may differ from the cross-section in the first longitudinal region 302. For example, the upper surface 602 may generally have a higher degree of curvature in the second longitudinal region 304 than in the first longitudinal region 302 through the radius of curvature of the upper surface 602 being smaller in the second longitudinal region 304. The cross-section in the third longitudinal region 306 may vary along the longitudinal axis 318 of the couch top 600 so as to provide a smooth transition between the first longitudinal region 302 and the second longitudinal region 304.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

The disclosure comprises the following items:
1. A radiotherapy couch top configured to support a subject thereon and comprising a curved lower surface.
2. The radiotherapy couch top according to item 1, comprising first and second lateral sides, wherein the curved lower surface forms a curved shape extending from the first lateral side to the second lateral side.
3. The radiotherapy couch top according to item 1 or item 2, wherein the curved lower surface is curved with respect to a lateral axis of the radiotherapy couch top.
4. The radiotherapy couch top according to item 3, wherein the curved lower surface is convex with respect to the lateral axis of the radiotherapy couch top.
5. The radiotherapy couch top according to any preceding item, wherein the curved lower surface is curved such that a lateral centre of the curved lower surface is at a lower vertical position than lateral edges of the curved lower surface.
6. The radiotherapy couch top according to any preceding item, wherein the curved lower surface is symmetrical with respect to a longitudinal axis of the radiotherapy couch top.
7. The radiotherapy couch top according to any preceding item, wherein the curved lower surface has a single, constant radius of curvature.
8. The radiotherapy couch top according to item 7, wherein the radius of curvature is in the range of 400 mm to 2000 mm.
9. The radiotherapy couch top according to any of items 1-6, wherein the curved lower surface comprises a plurality of sub-sections each with a different respective radius of curvature.
10. The radiotherapy couch top according to item 9, wherein the different respective radii of curvature are in the range of 200 mm to 2000 mm.
11. The radiotherapy couch top according to any preceding item, comprising a flat upper surface.
12. The radiotherapy couch top according to any of items 1-10, comprising a curved upper surface.
13. The radiotherapy couch top according to item 12, wherein the curved upper surface is curved such that a lateral centre of the curved upper surface is at a lower vertical position than lateral edges of the curved upper surface.
14. The radiotherapy couch top according to item 12 or item 13, wherein a curvature of the curved upper surface is different to a curvature of the curved lower surface.
15. The radiotherapy couch top according to any of items 12-14, wherein a thickness of the radiotherapy couch top between the curved upper surface and the curved lower surface varies laterally across the radiotherapy couch top.
16. The radiotherapy couch top according to any of items 12-15, wherein the curved upper surface has a radius of curvature in the range of 1500 mm to 2500 mm.
17. A patient support apparatus comprising:
   the radiotherapy couch top of any preceding item; and
   a patient support base configured to support the radiotherapy couch top.
18. A radiotherapy device comprising:
   a rotatable gantry;
   a radiation source;
   an imaging apparatus; and
   the patient support apparatus of item 17.

## Claims

1. A radiotherapy couch top configured to support a subject thereon and comprising:
a first lateral width in a first longitudinal region of the radiotherapy couch top;
a second lateral width in a second longitudinal region of the radiotherapy couch top, the second lateral width being smaller than the first lateral width; and
a curved lower surface.

2. The radiotherapy couch top according to claim 1, comprising first and second lateral sides, wherein the curved lower surface forms a curved shape extending from the first lateral side to the second lateral side.

3. The radiotherapy couch top according to claim 1 or claim 2, wherein the curved lower surface is curved with respect to a lateral axis of the radiotherapy couch top,
optionally wherein the curved lower surface is convex with respect to the lateral axis of the radiotherapy couch top.

4. The radiotherapy couch top according to any preceding claim, wherein the curved lower surface is curved such that a lateral centre of the curved lower surface is at a lower vertical position than lateral edges of the curved lower surface.

5. The radiotherapy couch top according to any preceding claim, wherein the curved lower surface is symmetrical with respect to a longitudinal axis of the radiotherapy couch top.

6. The radiotherapy couch top according to any preceding claim, wherein the curved lower surface has a single, constant radius of curvature,
optionally wherein the radius of curvature is in the range of 400 mm to 2000 mm.

7. The radiotherapy couch top according to any of claims 1-5, wherein the curved lower surface comprises a plurality of sub-sections each with a different respective radius of curvature,
optionally wherein the different respective radii of curvature are in the range of 200 mm to 2000 mm.

8. The radiotherapy couch top according to any preceding claim, wherein at least one of:
the first longitudinal region of the radiotherapy couch top is configured to support a head of the subject; and/or
the second longitudinal region of the radiotherapy couch top is configured to support a torso of the patient.

9. The radiotherapy couch top according to any preceding claim, comprising a third longitudinal region of the radiotherapy couch top between the first longitudinal region and the second longitudinal region, wherein the radiotherapy couch top has a third lateral width in the third longitudinal region which decreases from being equal to the first lateral width of the first longitudinal region at an end of the third longitudinal region which is adjacent to the first longitudinal region, to being equal to the second lateral width of the second longitudinal region at an end of the third longitudinal region which is adjacent to the second longitudinal region.

10. The radiotherapy couch top according to any preceding claim, wherein a curvature of the curved lower surface in the first longitudinal region is different to a curvature of the curved lower surface in the second longitudinal region,
optionally wherein a radius of curvature of the curved lower surface in the second longitudinal region is smaller than a radius of curvature of the curved lower surface in the first longitudinal region,
further optionally wherein the radius of curvature of the curved lower surface in the second longitudinal region is in the range of 250 mm to 1000 mm.

11. The radiotherapy couch top according to any preceding claim, comprising a flat upper surface.

12. The radiotherapy couch top according to any of claims 1-10, comprising a curved upper surface,
optionally wherein the curved upper surface is curved such that a lateral centre of the curved upper surface is at a lower vertical position than lateral edges of the curved upper surface.

13. The radiotherapy couch top according to claim 12, wherein at least one of:
A. a curvature of the curved upper surface is different to a curvature of the curved lower surface;
B. a thickness of the radiotherapy couch top between the curved upper surface and the curved lower surface varies laterally across the radiotherapy couch top; and/or
C. the curved upper surface has a radius of curvature in the range of 1500 mm to 2500 mm.

14. A patient support apparatus comprising:
the radiotherapy couch top of any preceding claim; and
a patient support base configured to support the radiotherapy couch top.

15. A radiotherapy device comprising:
a rotatable gantry;
a radiation source;
an imaging apparatus; and
the patient support apparatus of claim 14.
